Europäisches Patentamt

European Patent Office

Office européen des brevets

(19)

(11) EP 1 478 737 B1

(12) EUROPÄISCHE PATENTSCHRIFT

(45) Veröffentlichungstag und Bekanntmachung des Hinweises auf die Patenterteilung:
24.08.2005 Patentblatt 2005/34

(51) Int Cl.7: **C12N 5/06**, G01N 33/00, G01N 33/543, B01L 3/00

(21) Anmeldenummer: 03708128.8

(22) Anmeldetag: 24.02.2003

(86) Internationale Anmeldenummer:
PCT/EP2003/001871

(87) Internationale Veröffentlichungsnummer:
WO 2003/074683 (12.09.2003 Gazette 2003/37)

(54) **CMOS-PROZESS-KOMPATIBLE HOCH-DK-OBERFLÄCHENBESCHICHTUNG ZUR KAPAZITIVEN DETEKTION UND STIMULATION BIOLOGISCHER GEWEBE**

CMOS PROCESS-COMPATIBLE HIGH DIELECTRIC CONSTANT SURFACE COATING FOR THE DETECTION AND STIMULATION OF BIOLOGICAL TISSUE

REVETEMENT SUPERFICIEL A CONSTANTE DIELECTRIQUE ELEVEE COMPATIBLE AVEC LE PROCEDE CMOS POUR LA DETECTION ET LA STIMULATION CAPACITIVES DE TISSUS BIOLOGIQUES

(84) Benannte Vertragsstaaten:
**DE FR GB**

(30) Priorität: **01.03.2002 DE 10209075**

(43) Veröffentlichungstag der Anmeldung:
**24.11.2004 Patentblatt 2004/48**

(73) Patentinhaber: **Infineon Technologies AG
81669 München (DE)**

(72) Erfinder:
• **GABL, Reinhard
  80337 München (DE)**
• **BIRKENMAIER, Tamara
  81379 München (DE)**
• **EVERSMANN, Björn-Oliver
  80336 München (DE)**
• **FROMHERZ, Peter
  80686 München (DE)**
• **JENKNER, Martin
  82152 Planegg (DE)**
• **SCHREITER, Matthias
  81379 München (DE)**
• **THEWES, Roland
  82194 Gröbenzell (DE)**
• **WERSING, Wolfram
  83346 Bergen (DE)**

(74) Vertreter: **Müller-Boré & Partner Patentanwälte
Grafinger Strasse 2
81671 München (DE)**

(56) Entgegenhaltungen:
**WO-A-01/70002        DE-A- 10 032 568**

• **VASSANELLI S ET AL: "TRANSISTOR PROBES LOCAL POTASSIUM CONDUCTANCES IN THE ADHESION REGION OF CULTURED RAT HIPPOCAMPAL NEURONS" JOURNAL OF NEUROSCIENCE, NEW YORK, NY, US, Bd. 19, Nr. 16, 15. August 1999 (1999-08-15), Seiten 6767-6773, XP008006937 ISSN: 0270-6474**
• **SCHAETZTHAUER R ET AL: "NEURON-SILICON JUNCTION WITH VOLTAGE-GATED IONIC CURRENTS" EUROPEAN JOURNAL OF NEUROSCIENCE, OXFORD UNIVERSITY PRESS, GB, Bd. 10, Nr. 6, Juni 1998 (1998-06), Seiten 1956-1962, XP008006938 ISSN: 0953-816X**
• **VASSANELLI S ET AL: "Transistor records of excitable neurons FROM RAT BRAIN" APPLIED PHYSICS A: MATERIALS SCIENCE AND PROCESSING, SPRINGER VERLAG, BERLIN, DE, Bd. 66, 1998, Seiten 459-463, XP002210066 ISSN: 0947-8396**
• **VASSANELLI S ET AL: "Neurons from rat brain coupled to transistors" APPLIED PHYSICS A: MATERIALS SCIENCE & PROCESSING, SPRINGER INTERNATIONAL, DE, Bd. 65, 1997, Seiten 85-88, XP002210054 ISSN: 0947-8396**

- STRAUB BERNHARD ET AL: "Recombinant maxi-K channels on transistor, a prototype of iono-electronic interfacing" NATURE BIOTECHNOLOGY, NATURE PUBLISHING, US, Bd. 19, Nr. 2, Februar 2001 (2001-02), Seiten 121-124, XP002210053 ISSN: 1087-0156
- FROMHERZ P ET AL: "A NEURON-SILICON JUNCTION: A RETZIUS CELL OF THE LEECH ON AN INSULATED-GATE FIELD-EFFECT TRANSISTOR" SCIENCE, AMERICAN ASSOCIATION FOR THE ADVANCEMENT OF SCIENCE,, US, Bd. 252, 31. Mai 1991 (1991-05-31), Seiten 1290-1293, XP000574048 ISSN: 0036-8075
- STETT A ET AL: "TWO-WAY SILICON-NEURON INTERFACE BY ELECTRICAL INDUCTION" PHYSICAL REVIEW E. STATISTICAL PHYSICS, PLASMAS, FLUIDS, AND RELATED INTERDISCIPLINARY TOPICS, AMERICAN INSTITUTE OF PHYSICS, NEW YORK, NY, US, Bd. 55, Nr. 2, Februar 1997 (1997-02), Seiten 1779-1782, XP000913528 ISSN: 1063-651X
- FROMHERZ P: "INTERFACING NEURONS AND SILICON BY ELECTRICAL INDUCTION" BERICHTE DER BUNSEN-GESELLSCHAFT FUR PHYSIKALISCHE CHEMIE, VERLAG CHEMIE. WEINHEIM, DE, Bd. 100, Nr. 7, 1996, Seiten 1093-1102, XP000907082 ISSN: 0005-9021

**Beschreibung**

Die Erfindung betrifft einen Biochip zur kapazitiven Stimulation und/oder Detektion biologischer Gewebe gemäß Anspruch 1 sowie ein Verfahren zur Herstellung eines entsprechenden Biochips gemäß Anspruch 10.

Die unmittelbare Kommunikation zwischen Nervenzellen und elektrisch aktiven Festkörperstrukturen, wie z.B. Halbleitern, ist im Labormaßstab bereits seit einem guten Jahrzehnt keine Fiktion mehr. Erfolgreiche Laborversuche wurden beispielsweise von P. Fromherz et al. in Science 252, 1290 (1991); P. Fromherz et al. in Physical Review Letters 75, 1670 (1995) sowie P. Stett et al. in Physical Review E Vol. 55, No. 2, 1779 (1997) berichtet. Eine zusammenfassende Übersicht über die frühen Ergebnisse ist in P. Fromherz in Berichte der Bunsen Gesellschaft No. 7, 1093 (1996) enthalten. In jüngerer Zeit wurden ferner erste nach industriellen Maßstäben produzierte Biochips vorgestellt. Moderne Biochips eröffnen somit unterschiedlichste Einsatzgebiete von neurobiologischer Grundlagenforschung bis zu high-throughput-screening Anwendungen in der pharmazeutischen Industrie.

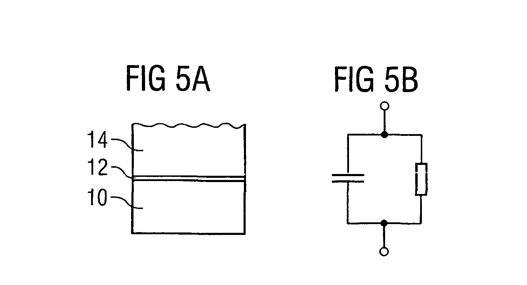

Ein Grundelement derartiger moderner Biochips ist schematisch in Fig. 5(a) dargestellt. Der Biochip umfaßt eine Trägerstruktur 10, welche beispielsweise ein strukturiertes Halbleitersubstrat umfassen kann (Halbleiterstruktur). Die Trägerstruktur 10 ist durch eine dielektrische Schicht 12 von einem Elektrolyten 14 getrennt ist. Das entsprechende Ersatzschaltbild ist in Fig. 5(b) dargestellt. Generell werden in einem Elektrolyten die elektrisch kommunizierenden Nervenzellen direkt an denjenigen Stellen auf der Oberfläche der Trägerstruktur 10 kultiviert, wo die aktiven Stellen elektrischer Stimulations- und Detektionseinrichtungen liegen.

Während in biologischen Systemen die elektrische Aktivität durch Ionen getragen wird, sind in Halbleitern Elektronen bzw. Löcher für den Ladungstransport zuständig. Entsprechend der nätürlichen Grenzschicht zwischen Elektrolyt 14 und Halbleiterstruktur wird deshalb sowohl bei der Stimulation als auch bei der Detektion biologischer Vorgänge vorzugsweise eine kapazitive Ankopplung ausgenutzt. Dieser stromfreie Mechanismus der elektrischen Kopplung zwischen dem Biochip und der zu stimulierenden bzw. zu detektierenden Nervenzelle basiert auf dem Prinzip der elektrischen Induktion (elektrostatische Induktion bzw. Influenz). Die elektrische Kopplung wird dadurch bewirkt, daß eine Ladungsakkumulation in der Nervenzelle entsprechende Spiegelladungen in dem Biochip induziert, deren Einfluß beispielsweise auf die elektrischen Transporteigenschaften der als Halbleiterstruktur ausgebildeten Trägerstruktur 10 nachgewiesen werden kann.

Umgekehrt lassen sich Nervenzellen, welche an der dielektrischen Schicht 12 der Träger- bzw. Halbleiterstruktur 10 angeordnet sind, dadurch stimulieren, daß eine Ladungsakkumulation in einer Stimulationseinrichtung der Halbleiterstruktur 10 Ladungen in der Nervenzelle induziert. Bei einem derartigen Biochip, welcher sowohl bei Stimulations- als auch bei Detektionsprozessen eine kapazitive Ankopplung zu der zu untersuchenden Nervenzelle ausnutzt, kommt der dielektrischen Schicht 12 zwischen der Halbleiterstruktur 10 und dem Elektrolyten 14 eine besondere Bedeutung zu.

Herkömmlicherweise werden siliziumbasierte, aktive und damit CMOS-fähige Halbleiterstrukturen mit $SiO_2$ beschichtet, um eine derartige dielektrische Grenz- bzw. Oberflächenschicht 12 auszubilden. Es hat sich jedoch gezeigt, daß insbesondere hinsichtlich der Ankopplungseffizienz bzw. des erzielbaren Signalübertrags zwischen Stimulations- und/oder Sensoreinrichtung des Biochips und dem zu untersuchenden biologischen Gewebe eine dielektrische Schicht aus $SiO_2$ nur bedingt zufriedenstellende Ergebnisse liefert.

Aufgabe der Erfindung ist es demgemäß, einen Biochip, welcher eine verbesserte Ankopplungseffizienz bzw, einen gesteigerten Signalübertrag zwischen Stimulations- und/oder Detektionseinrichtungen des Biochips und dem zu untersuchenden biologischen Gewebe ermöglicht, bereitzustellen. Ferner ist es Aufgabe der Erfindung, ein Herstellungsverfahren eines entsprechenden Biochips anzugeben.

Diese Aufgabe wird durch einen Biochip mit den in Anspruch 1 angegebenen Merkmalen sowie durch ein Verfahren zur Herstellung eines Biochips mit den in Anspruch 10 angegebenen Merkmalen gelöst. Bevorzugte Ausführungsformen sind Gegenstand der abhängigen Ansprüche.

Erfindungsgemäß umfaßt ein Biochip zur kapazitiven Stimulation und/oder Detektion biologischer Gewebe

- eine Trägerstruktur;
- zumindest eine Stimulations- und/oder Sensoreinrichtung, welche in bzw. an der Trägerstruktur angeordnet ist;
- zumindest eine dielektrische Schicht, deren eine Schichtfläche an der Stimulations- und/oder Sensoreinrichtung angeordnet ist und deren gegenüberliegende Schichtfläche eine Stimulations-und/oder Sensorfläche zur kapazitiven Stimulation und/oder Detektion biologischer Gewebe bildet;
- wobei die dielektrische Schicht $TiO_2$ umfaßt.

Gemäß der Erfindung umfaßt die dielektrische Schicht, welche betriebsmäßig zwischen der Trägerstruktur des Biochips und einem Elektrolyten mit dem zu untersuchenden biologischen Material angeordnet ist, $TiO_2$, wobei die dielektrische Schicht vorzugsweise im wesentlichen aus $TiO_2$ besteht. Die Trägerstruktur kann beispielsweise mit planarlithographischen Prozeßschritten, wie sie insbesondere aus der CMOS-Halbleitertechnik bekannt sind, aus einem Trägersubstrat hergestellt werden. Vorzugsweise werden die Stimulations-und/oder Sensoreinrichtung durch einen CMOS-Prozeß in einem Siliziumhalbleitersubstrat ausgebildet.

Überraschenderweise hat sich gezeigt, daß eine TiO$_2$ umfassende dielektrische Schicht wesentliche Vorteile im Vergleich zu herkömmlichen dielektrischen Grenzschichten aus SiO$_2$ aufweist. Insbesondere führt eine dielektrische Schicht mit TiO$_2$ im Vergleich zu einer SiO$_2$-Grenzschicht zu einer deutlich verbesserten Ankopplungseffizienz bzw. zu einem höherem Signalübertrag zwischen Biochip und biologischen Material. Folgende Vorteile eines erfindungsgemäßen Biochips mit einer dielektrischen Grenzschicht mit TiO$_2$ sind besonders hervorzuheben:

(a) Hohe spezifische Kapazität

Aufgrund der Beziehung

$$c = \frac{\varepsilon_r \varepsilon_0}{d},$$

wobei $c$ die spezifische Kapazität des "Grenzschichtkondensators" in F/m$^2$, $\varepsilon_0 = 8,85 \times 10^{-12}$ As/Vm die elektrische Feldkonstante, $\varepsilon_r$ die relative

Dielektrizitätskonstante und d die Dicke der dielektrischen Schicht in Meter ist, sollte die Schicht für eine hohe spezifische Kapazität zum einen möglichst dünn sein und zum anderen aus einem Material bestehen, welches eine große Dielektrizitätskonstante aufweist. Die gesamte Schicht sollte auch noch bei geringen Schichtdicken und in Kontakt mit unterschiedlichen Materialien eine derartige hohe Dielektrizitätskonstante aufweisen.

Experimentell konnte bei erfindungsgemäßen Biochips, welche TiO$_2$-Schichten als dielektrische Grenzschichten aufwiesen, Dielektrizitätskonstanten von 46 nachgewiesen werden. Derartige Dielektrizitätskonstanten liegen deutlich über denjenigen von SiO$_2$, Si$_3$Ni$_4$, Al$_2$O$_3$, ZrO$_2$, HfO$_2$, La$_2$O$_3$, Ta$_2$O$_5$ und Y$_2$O$_3$. Da die spezifische Kapazität der Trägerstruktur-Elektrolytgrenzfläche, welche durch die dielektrische Beschichtung der Trägerstruktur, beispielsweise einer Halbleiterstruktur, dominiert wird, ein wesentlicher Parameter bei der Optimierung der Ankopplungseffizienz bzw. des Signalübertrags ist, läßt sich durch die Wahl von TiO$_2$ eine wesentliche Verbesserung gegenüber herkömmlich verwendeten dielektrischen Grenzschichten erzielen.

(b) Leckleitfähigkeit

Die dielektrische Grenzschicht eines erfindungsgemäßen Biochips weist eine sehr geringe Leckleitfähigkeit auf, so daß elektrolytische Reaktionen an der Schichtoberfläche, welche mit Ohmschen Strömen einhergehen, ausgeschlossen sind.

(c) Stabilität und Korrosion

Im Gegensatz zu konventionellen Kondensatoren, die beidseitig von einem Festkörperkontakt begrenzt werden, muß der "Kondensatoraufbau" eines Biochips einer einseitigen Kontaktierung durch eine wässrige Lösung mit gelösten Ionen widerstehen. Insbesondere sollen sich auch unter Anlegen einer elektrischen Spannung die Eigenschaften der dielektrischen Grenzschicht nicht ändern. Ferner sollten Stoffwechselprodukte des biologischen Materials, beispielsweise einer Zellkultur, nicht zu einer chemischen Korrosion der dielektrischen Grenzfläche führen. Überraschenderweise wurde festgestellt, daß dielektrische Grenzschichten mit TiO$_2$ - im Gegensatz zu Si$_3$N$_4$-Schichten - eine hohe Stabilität und geringe Korrosionsneigung aufweisen.

(d) Biokompatibilität

Umgekehrt darf auch die dielektrische Grenzschicht, insbesondere unter elektrischer Spannung, weder durch Auflösung noch durch Korrosion Partikel in den Elektrolyten entlassen. Da die Wechselwirkungen zwischen der dielektrischen Grenzschicht und dem biologischen Gewebe komplexer Natur sind, ist letztendlich nur die Vitalität von Zellen auf der Schichtoberfläche ein Maß für die Biokompatibilität des aufgebrachten biologischen Materials. Unter Vitalität ist insbesondere der Erhalt der Wachstumsfähigkeit, welches die Ausbildung von Neuriten auf der Oberfläche ermöglicht, zu verstehen.

Überraschenderweise genügt eine dielektrische Schicht, welche TiO$_2$ aufweist, in hervorragender Weise auch dieser Anforderung. So konnte erfolgreich ein Wachstum von Zellstrukturen auf TiO$_2$-Schichten nachgewiesen werden. Dielektrische Schichten mit TiO$_2$ sind somit Oberflächenbeschichtungen überlegen, welche giftige Schwermetalle oder andere toxische Substanzen enthalten (wie beispielsweise PZT, welches Blei enthält).

(e) Oberflächenbeschaffenheit

Es hat sich gezeigt, daß eine dielektrische Schicht mit TiO$_2$ ferner eine besonders vorteilhafte Oberflächenbeschaffenheit aufweist. So geht in die Wachstumsfähigkeit von Zellen nicht nur die Zusammensetzung der Grenzschicht, sondern insbesondere auch die Topologie der Oberfläche ein, auf welcher die Zellen kultiviert werden. Vorzugsweise weist die Oberfläche eine lediglich schwach ausgeprägte Topologie auf. Insbesondere sollten die charakteristischen Größen der Oberflächenstruktur in ihren Ausmaßen um mehrere Größenordnungen unter denjenigen liegen, welche verwendet werden, um beispielsweise ein gerichtetes Zellwachstum zu verursachen. Für typische Zellen sollten die Oberflächenstrukturen somit kleiner als 10 nm sein. Derartig plane Oberflächen halten zusätzlich die Möglichkeit einer mikromechanischen oder photolithographischen Strukturierung offen.

Diesen Eigenschaften genügt eine dielektrische Schicht mit $TiO_2$ in hervorragender Weise. Ferner konnte gezeigt werden, daß das Wachstum einer Zellkultur keine nennenswerten Auswirkungen auf die Oberflächenstruktur einer derartigen dielektrischen Schicht hat. Sowohl vor als auch nach dem Wachstum der Zellkultur betrugen die Höhenschwankungen der dielektrischen Oberfläche lediglich einige Nanometer, so daß die Grenzoberfläche als glatt eingestuft werden kann.

(f) Reinigungsfähigkeit

Es hat sich gezeigt, daß derartig glatte dielektrische Schichten mit $TiO_2$ zusätzlich den Vorteil einer leichten Reinigungsfähigkeit bieten. Vorzugsweise sollten Oberflächen für das Wachstum von Zellkulturen nicht nur glatt, sondern auch für Reinigungslösungen vollständig zugänglich und leicht spülbar sein. Die dielektrische Schicht eines erfindungsgemäßen Biochips ist in einem weiten pH-Bereich widerstandsfähig, so daß übliche Reinigungslösungen für biologische Materialien, welche zum Teil extreme pH-Werte aufweisen, eingesetzt werden können.

Zur Reinigungsfähigkeit von Oberflächen gehört ferner auch deren Sterilisierbarkeit. Bei planaren Systemen eignet sich besonders eine UV-Sterilisierung, welche jedoch eine entsprechende UV-Stabilität voraussetzt. Vorteilhafterweise ist die UV-Stabilität von dielektrischen Schichten mit $TiO_2$ derartig ausgeprägt, daß erfindungsgemäße Biochips vor einem betriebsmäßigen Einsatz einer Sterilisationsbehandlung durch UV-Bestrahlung unterworfen werden können. Die UV-Festigkeit von $TiO_2$ Schichten stellt somit einen gewichtigen Vorteil gegenüber den meisten organischen Oberflächenbeschichtungen dar.

(g) Homogenität

Ferner konnte gezeigt werden, daß dielektrische Schichten mit $TiO_2$ mit ausreichender Homogenität herstellbar sind. Vorzugsweise sollten nämlich die zuvor beschriebenen Anforderungen an die dielektrische Schicht für die gesamte Stimulations-und/oder Sensorfläche erfüllt sein. Bereits kleine Gebiete mit hoher Schichtleitfähigkeit reichen beispielsweise aus, um den gewünschten Isolatorcharakter der gesamten Schicht zu zerstören. Ähnliches gilt für eine Verteilung der Dielektrizitätskonstanten bzw. für die Schichtdicke.

(h) CMOS-Integrabilität

Ein besonderer Vorteil von dielektrischen Grenzschichten mit $TiO_2$ ist deren CMOS-Integrabilität. Die Lage der dielektrischen Schicht in der obersten und damit zuletzt prozessierten Schicht eines CMOS-Prozesses bewirkt, daß bei ihrer Abscheidung auf die bereits vorhandenen Strukturen Rücksicht genommen werden muß. Das schränkt insbesondere den Temperaturbereich des eingesetzten Abscheidungsprozesses sowie die Dauer des Prozeßschritts ein. Der Abscheidungsprozeß der dielektrischen Schicht muß somit in das "Temperaturbudget" des Biochips im back-end-Prozeßstadium passen. Vorzugsweise sollte eine Maximaltemperatur von etwa $500°C$ nicht überschritten werden.

Überraschenderweise hat sich gezeigt, daß $TiO_2$-Schichten in einer Qualität, welche sämtliche obigen Eigenschaften erfüllt, beispielsweise mittels reaktivem RF-Sputtern bei Temperaturen um $400°C$ abgeschieden werden kann. Ein derartiger reaktiver RF-Sputterprozeß ist auch noch als back-end-Prozeß in einem CMOS-Herstellungsverfahren einsetzbar. Auch ein Sputterverfahren mit gepulsten DC-Quellen kann zum Einsatz kommen.

$TiO_2$-Schichten weisen ferner den Vorteil auf, daß deren Herstellung die bereits prozessierten Strukturen unterhalb der Oberflächenschicht auch nicht auf andere Art und Weise nachteilig verändert. Ferner kann die $TiO_2$-Schicht mit industriell üblichen Verfahren auf Halbleiterwafern üblicher Größe hergestellt werden.

Ein weiterer Vorteil einer dielektrischen Schicht mit $TiO_2$ liegt in ihrer hohen mechanischen Festigkeit. So konnte nachgewiesen werden, daß im Gegensatz zu $Al_2O_3$ bei der Vorbereitung der dielektrischen Grenzfläche eines erfindungsgemäßen Biochips auf einen Versuchseinsatz die aufgebrachte dielektrische Schicht nicht beschädigt oder abgerieben wurde.

Vorzugsweise umfaßt die Stimulationseinrichtung eine Metallelektrode, deren elektrisches Potential extern steuerbar ist, und die dielektrische Schicht ist an der Metallelektrode angeordnet ist.

Vorzugsweise umfaßt die Trägerstruktur eine Halbleiterstruktur. Die Halbleiterstruktur kann insbesondere eine Silizium-CMOS-Struktur sein.

Vorzugsweise umfaßt die Sensoreinrichtung einen Feldeffekttransistor mit einem Source-, einem Drain- und einem Gatekontakt.Bei dem Feldeffekttransistor kann es sich insbesondere um einen p- oder um einen n-Transistor handeln, welcher in dem "front-end" eines CMOS-Prozesses gebildet wird.

Vorzugsweise ist die dielektrische Schicht an einer Metallelektrode der Sensoreinrichtung angeordnet, welche elektrisch leitfähig mit dem Gatekontakt des Feldeffekttransistors verbunden ist. Bevorzugt ist die Halbleiterstruktur eine CMOS-Halbleiterstruktur. Insbesondere kann die Metallelektrode über eine Anordnung von Metall- und Intermetallschichten der CMOS-Halbleiterstruktur elektrisch leitfähig mit dem Gatekontakt verbunden sein. Während der Feldeffekttransistor der CMOS-Halbleiterstruktur im "front-end" definiert wurde, erfolgt die Anordnung der Metallelektrode und der $TiO_2$-Grenzschicht im "back-end", d.h. in einem spätem Prozeßstadium.

Die Sensoreinrichtung eines derartigen Biochips weist demgemäß zwei in Serie geschaltete Kapazitäten auf, welche die Ankopplungseffizienz beeinflussen. So weist die Sensoreinrichtung eine Grenzschichtkapazität auf, welche durch die Anordnung Elektrolyt - TiO$_2$-Grenzschicht - Metallelektrode definiert wird. Zusätzlich.geht in die Ankopplungseffizienz die Gatekapazität des MOSFETs ein, welche durch die Anordnung Metall bzw. Polysilizium - SiO$_2$-Halbleiter bestimmt wird. Eine gute Ankopplungseffizienz zwischen Biochip und Elektrolyt wird dann erzielt, wenn die Grenzschichtkapazität möglichst hoch ist.

Bei einer besonders einfach aufgebauten Ausführungsform eines erfindungsgemäßen Biochips kann die dielektrische TiO$_2$-Grenzschicht das Gateoxid des Feldeffekttransistors darstellen. Das Gateoxid des FETs bildet somit unmittelbar die dielektrische Grenzschicht zwischen Elektrolyt und Halbleiter. Der Gatekontakt, welcher ansonsten aus Metall oder Polysilizium besteht, wird in diesem Fall durch den Elektrolyten bereitgestellt. Ein derartiger Aufbau kann aufgrund seiner einfachen Herstellbarkeit als "Labormuster-Biochip" vorteilhaft sein.

Bevorzugt weist die dielektrische Schicht eine Schichtdicke zwischen 5 nm und 200 nm auf.

Gemäß der Erfindung umfaßt ein Verfahren zur Herstellung eines Biochips, insbesondere eines erfindungsgemäßen Biochips, zur kapazitiven Stimulation und/oder Detektion biologischer Gewebe die Schritte:

- Bereitstellen einerTrägerstruktur;
- Ausbilden zumindest einer Stimulations- und/oder einer Sensoreinrichtung in bzw. an der Trägerstruktur;
- Anordnen einer dielektrischen Schicht an der Stimulations- und/oder Sensoreinrichtung derart, daß eine Schichtfläche der dielektrischen Schicht an der Stimulations- und/oder Sensoreinrichtung angeordnet ist und die gegenüberliegende Schichtfläche der Schicht eine Stimulations- und/oder Sensorfläche zur kapazitiven Stimulation und/oder Detektion biologischer Gewebe bildet;

wobei die dielektrische Schicht TiO$_2$ umfaßt.

Vorzugsweise umfaßt der Schritt des Anordnens der dielektrischen Schicht Sputtern von TiO$_2$. Bevorzugt erfolgt das Sputtern von einem metallischen Titantarget in einem Argon/Sauerstoffgemisch. Bevorzugt erfolgt der Schritt des Anordnens der dielektrischen Schicht am Ende (d.h. im "back-end") eines CMOS-Prozesses.

Die Erfindung wird nachfolgend mit Bezug auf begleitende Zeichnungen bevorzugter Ausführungsformen beispielhaft beschrieben. Es zeigt:

Fig. 1(a)  Eine Ausführungsform eines erfindungsgemäßen Biochips in einer Feldeffekttransistorbeschaltung als Potentialsensor;

Fig. 1(b)  eine weitere Ausführungsform eines erfindungsgemäßen Biochips in einer Stimulationsbeschaltung;

Fig. 2(a)  eine rasterkraftmikroskopische Aufnahme einer TiO$_2$-Schicht einer Ausführungsform eines erfindungsgemäßen Biochips vor dem Kontakt mit einem zu untersuchenden biologischen Material;

Fig. 2(b)  eine rasterkraftmikroskopische Aufnahme der Oberflächenbeschaffenheit und des Oberflächenprofils der TiO$_2$-Schicht von Fig. 2(a) nach einer 11-tägigen Kultivierung von fötalen Rattenhirnschnitten;

Fig. 3  eine Aufsicht auf eine dielektrische Schicht aus TiO$_2$ einer Ausführungsform eines erfindungsgemäßen Biochips, auf welcher gut wachsende HEK-Zellen angeordnet sind;

Fig. 4  ein Spannungs-Zeit-Diagramm eines Stimulationsexperimentes an einer vereinzelten Invertebratenzelle unter Verwendung einer Ausführungsform eines erfindungsgemäßen Biochips; und

Fig. 5  eine Grundstruktur der betriebsmäßigen Anordnung eines Biochips relativ zu einem Elektrolyten mit zugehörigem Ersatzschaltbild.

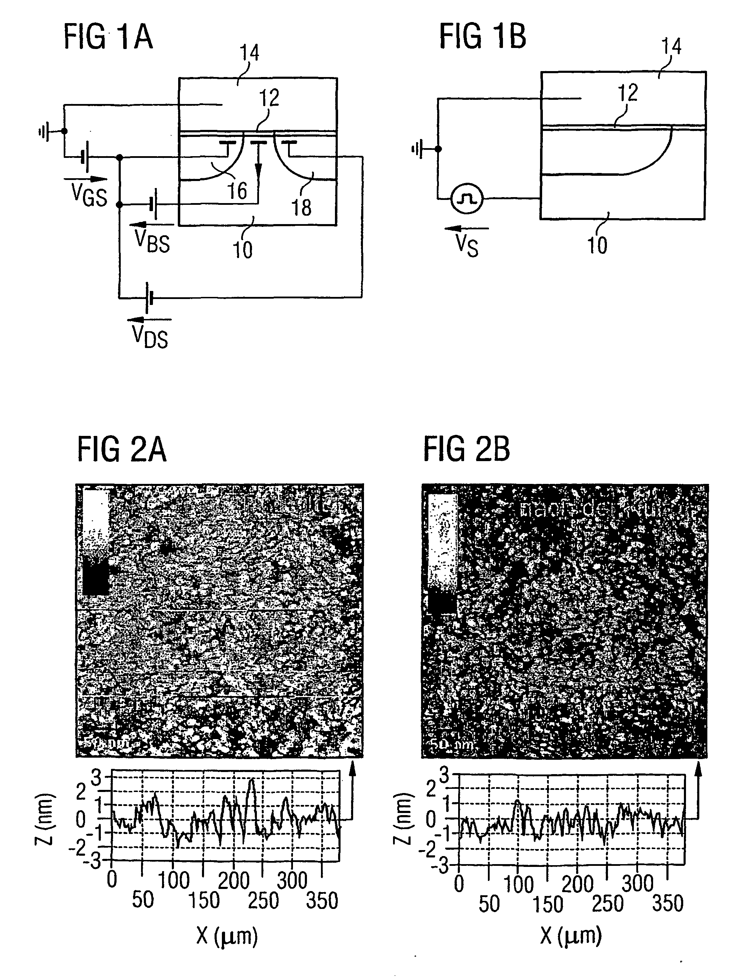

In Fig. 1(a) ist eine Ausführungsform eines erfindungsgemäßen Biochips in Feldeffekttransistorbeschaltung dargestellt, welcher als Potentialsensor verwendet wird. Der Biochip weist eine strukturierte Trägerstruktur 10 auf, bei welcher es sich um eine Halbleiterstruktur handelt.Das Substrat der Halbleiterstruktur 10 ist mittels eines ohmschen Kontaktes elektrisch kontaktiert und mit einer Spannungsquelle V$_{BS}$ elektrisch verbunden. In der Halbleiterstruktur 10 ist ein Sourcekontakt 16 sowie ein Drainkontakt 18 eines Feldeffekttransistors angeordnet, welche jeweils elektrisch kontaktiert sind.

In dem Gatebereich des Feldeffekttransistors zwischen dem Sourcekontakt 16 und dem Drainkontakt 18 ist eine dielektrische Schicht mit TiO$_2$ angeordnet, welche die Halbleiterstruktur 10 von dem Elektrolyten bzw. dem Bad 14 trennt. Die dielektrische Schicht 12 stellt somit das Gateoxid des Feldeffekttransistors dar.

Es handelt sich somit bei der in Fig. 1 dargestellten Ausführungsform um einen "Labormuster-Biochip", welcher einen besonders einfachen Aufbau aufweist. Ein erfindungsgemäßer CMOS-kompatibler Biochip weist einen wesentlich komplexeren Aufbau der Halbleiterstruktur 10 auf. Insbesondere wird bei einem CMOS-kompatiblen Biochip das Gateoxid des Feldeffekttransistors (FET) nicht durch die dielektrische TiO$_2$-Grenzschicht gebildet. Statt dessen weist der FET eines CMOS-kompatiblen Biochips einen herkömmlichen Aufbau mit einem Polysiliziumgatekontakt und einem SiO$_2$-Gateoxid auf. Der Gatekontakt ist in diesem Fall über eine Anordnung von Metall- und Intermetallbahnen mit einer oberflächennahen Metallelektrode verbunden, welche die dielektrische Grenzschicht aus $TiO_2$ trägt. Die weitere Beschreibung erfolgt jedoch aus Gründen der einfacheren Verständlichkeit des erfindungsgemäßen Grundprinzips anhand eines "Labormuster-Biochips".

Die dem Halbleitersubstrat 10 abgewandte Oberfläche der dielektrischen Schicht 12, d.h. derjenigen Grenzfläche, welche betriebsmäßig mit dem Elektrolyten 14 in Berührung tritt, stellt eine Sensorfläche zur kapazitiven Detektion biologischer Gewebe bzw. Vorgänge dar. Vereinfacht ausgedrückt, wird bei einem "Labormuster-Biochip" die herkömmlicherweise metallische Gateelektrode des Feldeffekttransistors durch den Elektrolyten ersetzt. Ladungsverschiebungen bzw. elektrische Potentialänderungen in dem biologischen Material, welches sich nahe der dielektrischen Schicht 12 in dem Elektrolyten 14 befindet, verändern somit über den Feldeffekt die elektrischen Transporteigenschaften des Feldeffekttransistors. Insbesondere wird die elektrische Leitfähigkeit zwischen dem Sourcekontakt 16 und dem Drainkontakt 18 durch derartige Potentialänderungen beeinflußt. Diese Kanalleitfähigkeitsänderung des Feldeffekttransistors kann in üblicher Weise nachgewiesen werden. Die dielektrische Schicht 12 kann auch eine größere laterale Ausdehnung als der Kanalbereich des Feldeffekttransistors aufweisen.

In Fig. 1(b) ist eine Ausführungsform eines erfindungsgemäßen Biochips in Stimulationsbeschaltung dargestellt. Eine Stimulationseinrichtung, welche beispielsweise aus einem dotierten Halbleiterbereich der Halbleiterstruktur 10 oder einer Metallelektrode besteht, ist elektrisch kontaktiert, so daß relativ zu dem elektrischen Potential des Elektrolyten ein externes Spannungssignal angelegt werden kann. Zwischen der Stimulationseinrichtung und dem Elektrolyten 14 ist eine dielektrische Schicht 12 mit $TiO_2$ angeordnet. Die der Stimulationseinrichtung abgewandte Grenzfläche der dielektrischen Schicht 12 stellt eine Stimulationsfläche zur kapazitiven Stimulation biologischer Gewebe dar. Diese kapazitive Stimulationsfläche, welche auch als Reizspot bezeichnet wird, gestattet die kapazitive Anregung von zu untersuchendem biologischen Material. Die dielektrische Schicht kann auch eine laterale Ausdehnung über die Stimulationseinrichtung hinaus aufweisen.

Nachfolgend wird eine Ausführungsvariante des erfindungsgemäßen Herstellungsverfahrens dargestellt. Die dielektrische Schicht wird auf der Stimulations- und/oder Sensoreinrichtung mittels eines Sputterverfahrens von $TiO_2$ aufgebracht. Hierbei wird von einem metallischen Titantarget in einem Argon/Sauerstoffgemisch bei 0,4 Pa und 60 mm Abstand die $TiO_2$-Schicht aufgesputtert. Das zerstäubte Titanmaterial reagiert mit den $O_2$-Molekülen im Gasraum zu $TiO_2$. Alternativ zu einem RF-Sputterverfahren kann auch ein Sputterverfahren mit einer gepulsten DC-Quelle eingesetzt werden, welche eine äquivalente DC-Leistung von 3kW aufweist. Die auf etwa 400°C geheizten strukturierten Siliziumsubstrate (Wafer) rotieren unter dem Target, so daß das $TiO_2$ mit einer mittleren Rate von 2 nm/min aufgebracht wird. Bei allen Wafern wird unmittelbar vor der Herstellung ein kurzer Ätzschritt in HF durchgeführt (sogenannter HF dip-etch).

Es hat sich gezeigt, daß derartige $TiO_2$-Schichten ein 10-minütiges Bad bei 80°C unter Ultraschalleinfluß in einer Reinigungslösung, welche unter dem Markennamen "Ticopur" vertrieben wird, überstehen, ohne daß Verschlechterungen der Grenzflächeneigenschaften feststellbar ist. Die Oberflächenschicht der dielektrischen Schicht wird hierbei um weniger als 1 nm abgetragen.

Zum Test der Korrosionsbeständigkeit wurden die erhaltenen Biochips in neurobiologisch häufig verwendeten Kunststoff-Petrischalen (Typ "Falcon 3001") angeordnet und von beiden Seiten jeweils 30 Minuten durch UV-Bestrahlung in Flowboxen sterilisiert.

Die Beschichtung erfolgte mit 2 ml PBS (phosphate buffer saline) und 40 µl Collagen je Schale, welches aufpipetiert über Nacht einwirkte. Am folgenden Tag wurden die Falcon-Schalen einmal mit Reinstwasser (Millipore) gespült und mit einem Nährmedium befüllt (13,89 g DMEM, 3,7 g $NaHCO_3$, 1 l Wasser mit pH 6,8 (HCl oder NaOH)). Das Medium wurde steril filtriert und mit 500 µl L-Glutamin, 5,5 ml Penicillin/Streptomycin (Antibiotika) und 100 ml FKS (fötales Kälberserum) versetzt.

Die in "Falcon 3001"-Petrischalen kultivierten HEK-Zellen wurden aus einer Schale abpipetiert, im Verhältnis 1:4 auf die Biochips im Nährmedium aufgetropft und im Brutschrank bei 37°C und 5% $CO_2$ aufbewahrt. Nach 24, 36 und 72 Stunden wurden Mikroskopaufnahmen vorgenommen, um den Zustand der Zellen und des Biochips zu überprüfen.

Alle Zellen zeigten unabhängig vom Untergrund eine ähnliche Zellteilungsrate und wirkten im Vergleich zu dielektrischen Schichten aus $SiO_2$ unbeeinflußt. Daraus läßt sich schließen, daß sich bei allen untersuchten Materialien einerseits keine Substanzen ablösten, welche sich negativ auf die Zellkultur auswirkten und andererseits die Morphologie der Grenzflächenoberfläche prinzipiell für Zellkulturen geeignet ist.

Fig. 2(a) zeigt eine kraftmikroskopische Aufnahme sowie der Oberflächenbeschaffenheit und des Oberflächenprofils einer dielektrischen Schicht aus $TiO_2$ vor einer Belegung mit biologischem Material. In Fig. 2(b) ist die Oberflächenbeschaffenheit und das Oberflächenprofil der in Fig. 2(a) gezeigten Grenzfläche nach einer 11-tägigen Kultivierung von fötalem Rattenhirnschnitten dargestellt. Die 11-tägige Zellkultur hatte keine nennenswerten Auswirkungen auf die Oberflächenmorphologie. Die gesamte Oberfläche zeigt wie zu Beginn des Versuchs Höhenschwankungen im Nanometerbereich und kann somit als glatt eingestuft werden.

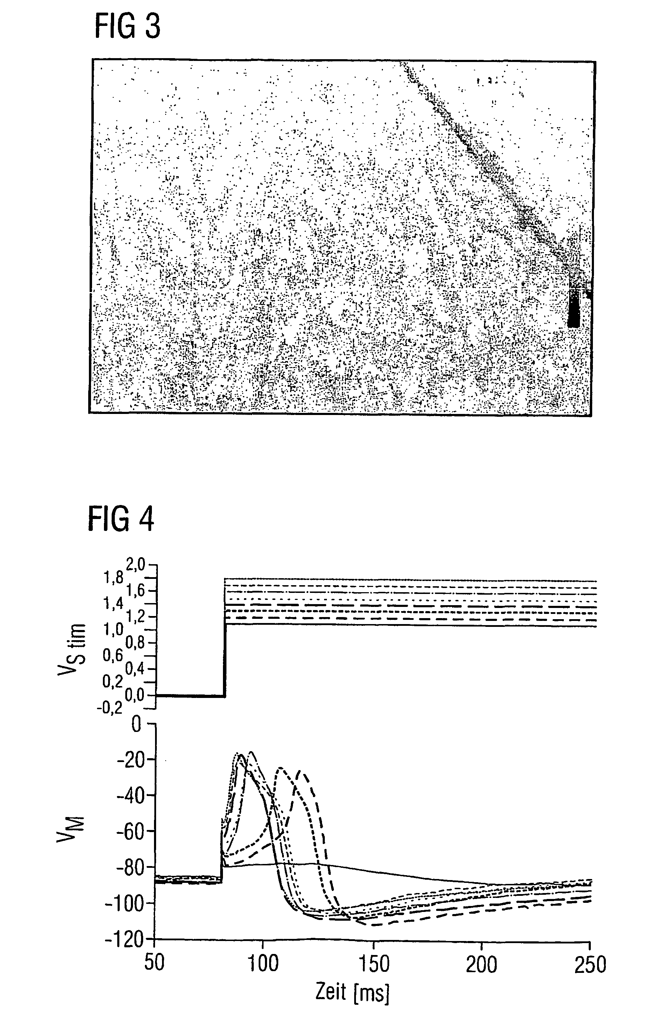

In Fig. 3 ist ein Biochip mit einer dielektrischen

Schicht aus $TiO_2$ dargestellt, welche mit einer gut wachsenden Schicht von HEK-Zellen belegt ist. Hieraus läßt sich ableiten, daß die dielektrische $TiO_2$-Schicht biokompatibel ist, da ein erfolgreiches Wachstum von HEK (human embryonic kidney) - Zellen erzielt werden konnte.

In Fig 4 ist ein Stimulationsexperiment mit einer weiteren Ausführungsform eines erfindungsgemäßen Biochips an einer vereinzelten Invertebratenzelle dargestellt. Neuronen von Lymnaea stagnalis werden kapazitiv mit einer Stimulationseinrichtung des Biochips stimuliert, in dem elektrische Spannungssprünge (oberes Diagramm von Fig. 4) zwischem dem unter der Neuron liegenden Halbleiterstruktur und dem Elektrolyt angelegt werden. Über den kapazitiven Spannungsteiler Dielektrikum-Zellmembran fällt kurzfristig eine depolarisierende Spannung über die Zellwand (unteres Diagramm) ab, welche oberhalb einer bestimmten Schwelle im Neuron ein Aktionspotential auslöst. Wesentlich ist, daß diese Schwelle bei der gegebenen Dicke der $TiO_2$-Schicht bei ungefähr einem Volt und damit ungefähr bei einem Drittel der Höhe entsprechender dielektrischer Schichten aus $SiO_2$ liegt. Die Ankopplungseffizienz der dielektrischen Schicht aus $TiO_2$ an das biologische Material ist somit im Vergleich zu herkömmlichen $SiO_2$-Schichten wesentlich verbessert.

**Bezugszeichenliste**

10    Trägerstruktur, insbesondere Halbleiterstruktur
12    dielektrische Schicht
14    Elektrolyt mit dem zu untersuchenden biologischen Material
16    Sourcekontakt
18    Drainkontakt

**Patentansprüche**

1.  Biochip zur kapazitiven Stimulation und/oder Detektion biologischer Gewebe (14) mit

    -    einer Trägerstruktur (10);
    -    zumindest einer Stimulations- und/oder einer Sensoreinrichtung, welche in bzw. an der Trägerstruktur (10) angeordnet ist;
    -    zumindest einer dielektrischen Schicht (12), deren eine Schichtfläche an der Stimulations- und/oder Sensoreinrichtung angeordnet ist und deren gegenüberliegende Schichtfläche eine Stimulations- und/oder Sensorfläche zur kapazitiven Stimulation und/oder Detektion biologischer Gewebe (14) bildet;

    wobei die dielektrische Schicht (12) $TiO_2$ umfaßt.

2.  Biochip nach Anspruch 1, wobei die Stimulationseinrichtung eine Metallelektrode umfaßt, deren elektrisches Potential extern steuerbar ist, und die dielektrische Schicht (12) an der Metallelektrode angeordnet ist.

3.  Biochip nach Anspruch 1 oder 2, wobei die Trägerstruktur (10) eine Halbleiterstruktur umfaßt.

4.  Biochip nach Anspruch 3, wobei die Sensoreinrichtung einen Feldeffekttransistor mit einem Source-(16), einem Drain-(18) und einem Gatekontakt umfaßt.

5.  Biochip nach Anspruch 4, wobei die dielektrische Schicht (12) an einer Metallelektrode der Sensoreinrichtung angeordnet ist, welche elektrisch leitfähig mit dem Gatekontakt des Feldeffekttransistors verbunden ist.

6.  Biochip nach einem der Ansprüche 3 bis 5, wobei die Halbleiterstruktur eine CMOS-Halbleiterstruktur ist.

7.  Biochip nach Anspruch 5 und 6, wobei die Metallelektrode über eine Anordnung von Metall- und Intermetallschichten der CMOS-Halbleiterstruktur elektrisch leitfähig mit dem Gatekontakt verbunden ist.

8.  Biochip nach Anspruch 3, wobei die dielektrische Schicht (12) das Gateoxid eines Feldeffekttransistors bildet.

9.  Biochip nach einem der vorangegangenen Ansprüche, wobei die dielektrische Schicht (12) eine Schichtdicke zwischen 5 nm und 200 nm aufweist.

10. Verfahren zur Herstellung eines Biochips zur kapazitiven Stimulation und/oder Detektion biologischer Gewebe (14) mit den Schritten:

    -    Bereitstellen einer Trägerstruktur (10);
    -    Ausbilden zumindest einer Stimulations- und/ oder einer Sensoreinrichtung in bzw. an der Trägerstruktur (10);
    -    Anordnen einer dielektrischen Schicht (12) an der Stimulations- und/oder Sensoreinrichtung derart, daß eine Schichtfläche der dielektrischen Schicht (12) an der Stimulations- und/ oder Sensoreinrichtung angeordnet ist und die gegenüberliegende Schichtfläche der Schicht (12) eine Stimulations- und/oder Sensorfläche zur kapazitiven Stimulation und/oder Detektion biologischer Gewebe (14) bildet;

    wobei die dielektrische Schicht (12) $TiO_2$ umfaßt.

**11.** Verfahren nach Anspruch 10, wobei der Schritt des Anordnens der dielektrischen Schicht (12) Sputtern von TiO$_2$ umfaßt.

**12.** Verfahren nach Anspruch 11, wobei das Sputtern von einem metallischen Titantarget in einem Argon/ Sauerstoffgemisch erfolgt.

**13.** Verfahren nach Anspruch 11 oder 12, wobei der Schritt des Anordnens RF-Sputtern oder Sputtern mit einer gepulsten DC-Quelle umfaßt.

**14.** Verfahren nach Anspruch 10 bis 13, wobei der Schritt des Anordnens der dielektrischen Schicht (12) als back-end Prozeßschritt eines CMOS-Prozesses erfolgt.

**Claims**

**1.** Biochip for capacitive stimulation and/or detection of biological tissues (14) having

- a carrier structure (10);
- at least one stimulation and/or sensor device, which is arranged in or at the carrier structure (10);
- at least one dielectric layer (12), one layer area of which is arranged at the stimulation and/or sensor device and the opposite layer of which forms a stimulation and/or sensor area for capacitive simulation and/or detection of biological tissues (14);

the dielectric layer (12) comprising TiO$_2$.

**2.** Biochip according to Claim 1, the stimulation device comprising a metal electrode, the electrical potential of which can be controlled externally, and the dielectric layer (12) being arranged at the metal electrode.

**3.** Biochip according to Claim 1 or 2, the carrier structure (10) comprising a semiconductor structure.

**4.** Biochip according to Claim 3, the sensor device comprising a field-effect transistor having a source contact (16), a drain contact (18) and a gate contact.

**5.** Biochip according to Claim 4, the dielectric layer (12) being arranged at a metal electrode of the sensor device, which is electrically conductively connected to the gate contact of the field-effect transistor.

**6.** Biochip according to one of Claims 3 to 5, the semiconductor structure being a CMOS semiconductor structure.

**7.** Biochip according to Claims 5 and 6, the metal electrode being electrically conductively connected to the gate contact via an arrangement of metal and intermetal layers of the CMOS semiconductor structure.

**8.** Biochip according to Claim 3, the dielectric layer (12) forming the gate oxide of a field-effect transistor.

**9.** Biochip according to one of the preceding claims, the dielectric layer (12) having a layer thickness of between 5 nm and 200 nm.

**10.** Method for producing a biochip for capacitive stimulation and/or detection of biological tissues (14), having the following steps:

- provision of a carrier structure (10);
- formation of at least one stimulation and/or sensor device in or at the carrier structure (10);
- arrangement of a dielectric layer (12) at the stimulation and/or sensor device in such a way that one layer area of the dielectric layer (12) is arranged at the stimulation and/or sensor device and the opposite layer area of the layer (12) forms a stimulation and/or sensor area for capacitive stimulation and/or detection of biological tissues (14);

the dielectric layer (12) comprising TiO$_2$.

**11.** Method according to Claim 10, the step of arrangement of the dielectric layer (12) comprising sputtering of TiO$_2$.

**12.** Method according to Claim 11, the sputtering of a metallic titanium target being effected in an argon/ oxygen mixture.

**13.** Method according to Claim 11 or 12, the arrangement step comprising RF sputtering or sputtering by means of a pulsed DC source.

**14.** Method according to Claims 10 to 13, the step of arrangement of the dielectric layer (12) being effected as a back end process step of a CMOS process.

**Revendications**

**1.** Biopuce pour la stimulation et/ou la détection capacitives de tissus biologiques (14) avec :

- une structure porteuse (10);
- au moins un dispositif de stimulation et/ou de détection qui est agencé dans ou sur la structure porteuse (10);

- au moins une couche diélectrique (12) dont une surface de couche est agencée sur le dispositif de stimulation et/ou de détection et dont la surface de couche opposée forme une surface de stimulation et/ou de détection pour la stimulation et/ou la détection capacitives de tissus biologiques (14); la couche diélectrique (12) comprenant du $TiO_2$.

2. Biopuce selon la revendication 1, dans laquelle le dispositif de stimulation comprend une électrode métallique dont le potentiel électrique peut être réglé de l'extérieur et la couche diélectrique (12) est agencée sur l'électrode métallique.

3. Biopuce selon la revendication 1 ou 2, dans laquelle la structure porteuse (10) comprend une structure semi-conductrice.

4. Biopuce selon la revendication 3, dans laquelle le dispositif de détection comprend un transistor à effet de champ avec un contact de source (16), un contact de drain (18) et un contact de grille.

5. Biopuce selon la revendication 4, dans laquelle la couche diélectrique (12) est agencée sur une électrode métallique du dispositif de détection qui est reliée de manière électro-conductrice avec le contact de grille du transistor à effet de champ.

6. Biopuce selon l'une quelconque des revendications 3 à 5, dans laquelle la structure semi-conductrice est une structure semi-conductrice CMOS.

7. Biopuce selon les revendications 5 et 6, dans laquelle l'électrode métallique est reliée de manière électro-conductrice avec le contact de grille par un agencement de couches métalliques et intermétalliques de la structure semi-conductrice CMOS.

8. Biopuce selon la revendication 3, dans laquelle la couche diélectrique (12) forme l'oxyde de grille d'un transistor à effet de champ.

9. Biopuce selon l'une quelconque des revendications précédentes, dans laquelle la couche diélectrique (12) présente une épaisseur de couche située entre 5 nm et 200 nm.

10. Procédé pour la fabrication d'une biopuce pour la stimulation et/ou la détection capacitives de tissus biologiques (14) avec les étapes suivantes :

    - fabrication d'une structure porteuse (10);
    - agencement d'au moins un dispositif de stimulation et/ou de détection dans ou sur la structure porteuse (10);
    - agencement d'une couche diélectrique (12) sur le dispositif de stimulation et/ou de détection de telle sorte qu'une surface de couche de la couche diélectrique (12) est agencée sur le dispositif de stimulation et/ou de détection et que la surface de couche opposée de la couche diélectrique (12) forme une surface de stimulation et/ou de détection pour la stimulation et/ou la détection capacitives de tissus biologiques (14);

la couche diélectrique (12) comprenant du $TiO_2$.

11. Procédé selon la revendication 10, dans lequel l'étape d'agencement de la couche diélectrique (12) comprend la pulvérisation de $TiO_2$.

12. Procédé selon la revendication 11, dans lequel la pulvérisation d'une cible de titane métallique se fait dans un mélange argon/oxygène.

13. Procédé selon la revendication 11 ou 12, dans lequel l'étape d'agencement comprend une pulvérisation RF ou une pulvérisation avec une source CC pulsée.

14. Procédé selon les revendications 10 à 13, dans lequel l'étape d'agencement de la couche diélectrique (12) se passe comme l'étape de traitement finale d'un procédé CMOS.

## FIG 1A

## FIG 1B

## FIG 2A

## FIG 2B

## FIG 3

## FIG 4

## FIG 5A

14 —
12 —
10 —

## FIG 5B